# EUROPEAN PATENT APPLICATION

(11) **EP 4 111 982 A1**
(43) Date of publication of application: **04.01.2023**
(21) Application number: 21290044.3
(22) Date of filing: 29.06.2021
(51) Int. Cl.: A61B 8/00, A61B 8/08, A61B 8/12

(54) **SYSTEMS AND APPARATUSES FOR NAVIGATION AND PROCEDURAL GUIDANCE OF LASER LEAFLET RESECTION UNDER INTRACARDIAC ECHOCARDIOGRAPHY**

(71) Applicant: Koninklijke Philips N.V., 5656 AG Eindhoven (NL)
(72) Inventor: Pai Raikar, Vipul, 5656 AE Eindhoven (NL); Vaidya, Kunal, 5656 AE Eindhoven (NL); Anderson, Michael, 5656 AE Eindhoven (NL); Francis, Natan C, 5656 AE Eindhoven (NL); Mory, enoit Jean-Dominique Bertrand Maurice, 5656 AE Eindhoven (NL); Cathier, Pascal Yves François, 5656 AE Eindhoven (NL)
(74) Representative: Philips Intellectual Property & Standards

(57) **Abstract**

Systems and apparatuses for a resection procedure are provided. The apparatus includes an Intracardiac Echocardiography (ICE) probe for use in a Transcatheter Aortic Valve Replacement (TAVR) procedure, the ICE probe; and a processor device coupled to the ICE probe to provide positional feedback to a user about the ICE probe position as the ICE probe is positioned manually within cardiac anatomy wherein the processor device is configured to implement a model to provide guidance to manually position the ICE probe based on anatomical recognition of the cardiac anatomy wherein a manually positioned ICE probe is at located at a position in the cardiac anatomy to enable capture of a view of a target cardiac anatomy in combination with the use of a catheter used while performing the TAVR procedure.

## Description

### TECHNICAL FIELD

The technical field generally relates to endovascular procedures and more particularly relates to systems and apparatuses for imaging, image-guided navigation, and recognition of cardiac anatomy while performing a resection using a laser energy device under intracardiac echocardiography.

### BACKGROUND

The use of Machine Learning (ML) applications are becoming more prevalent and recognized for applicability in various surgical phases; with advancements in ML application potential new applications are feasible such as the use in anatomical recognition in surgical procedures with different intraoperative data inputs such as video and instrument type to assistant a medical provider in a surgical procedure.

Structural heart disease (SHD) is one of the fastest-growing fields in interventional radiology space which is a relatively new sub-specialty of interventional cardiology. The use of ML applications can play a part in this sub-specialty field to establish improvements in the standard of care for cardiac pathologies such as in the valvular disease domain.

Valve-in-valve repair has become deemed acceptable as a treatment for patients who have received a prior bio-prosthetic implant to prevent future valve failures and to mitigate the risk of complications such as coronary obstruction. From an imaging and device implantation perspective, the standard of care has migrated to performing these procedures purely on interventional x-ray fluoroscopy in a catheter lab environment. As these procedure numbers keep increasing and reinterventions become necessary, the impact of potential risks associated with reinterventions is an issue. During the procedure interventional x-ray alone is insufficient for monitoring of adverse events, such as pericardial effusion, which can lead to potentially fatal outcomes. Also, a vital necessary component of the imaging is the live visualization of the valve anatomy, particularly the leaflets. This imaging can currently be done only with some form of live ultrasound imaging. While typically, in structural heart procedures, live transoesophageal (TEE) ultrasound is used, there is an effort to move away from this as the need for general anaesthesia is eliminated. Transthoracic ultrasound (TTE) is also used but finding the right views can be challenging and highly dependent on patient anatomy as well as operator skill. Intracardiac echocardiography (ICE) has the potential to provide live ultrasound guidance and overcome the challenges of its traditional counterparts.

However, the use of current medical instruments that utilize cutting mechanisms presents certain drawbacks that when implemented intravascularly to successfully utilize ICE for this procedure. A newer methodology is required to enhance a safe user experience for both the patient and the non-traditional user of ultrasound imaging (i.e., the interventional radiologist). The cardiac cycle and rhythm introduce various types of motion which can affect the stability of the device as well as the images it produces. For a procedure that requires accurate and precise maneuvering of therapeutic devices, it is important to provide as much stability in the field of view as possible. In addition, for a non-traditional user of ultrasound, guidance, and feedback of relevance in both ultrasound as well as x-ray imaging can assist in reducing the cognitive load.

It is desirable to implement a system to stabilize the ICE probe, methods to provide navigation guidance and device trajectories with respect to key anatomical landmarks, and a method to successfully analyze the interaction of the resectioning device with its intended target, the valve leaflet.

The following disclosure provides these technological enhancements, in addition to addressing related issues.

### BRIEF SUMMARY

This summary is provided to describe select concepts in a simplified form that are further described in the Detailed Description. This summary is not intended to identify key or essential features of the claimed subject matter, nor is it intended to be used as an aid in determining the scope of the claimed subject matter.

The various embodiment describes apparatuses and systems to stabilize the ICE probe, to provide navigation guidance and device trajectories with respect to key anatomical landmarks, and to analyze the interaction of the resection device with an intended target, the valve leaflet.

In one exemplary embodiment, an apparatus including an Intracardiac Echocardiography (ICE) probe for use in a Transcatheter Aortic Valve Replacement (TAVR) procedure is provided. The apparatus includes the ICE probe, and a processor device coupled to the ICE probe to provide positional feedback to a user about the ICE probe position as the ICE probe is positioned manually within cardiac anatomy; wherein the processor device is configured to implement a model to provide guidance to manually position the ICE probe based on anatomical recognition of the cardiac anatomy wherein a manually positioned ICE probe is at located at a position in the cardiac anatomy to enable capture of a view of a target cardiac anatomy in combination with the use of a catheter used while performing the TAVR procedure.

In at least one exemplary embodiment, an apparatus that is provided includes the processor device that is further configured to determine an initial position and a three-dimensional (3D) orientation of the ICE probe within the cardiac anatomy to display a field of view (FOV) of the cardiac anatomy while performing the TAVR procedure.

In at least one exemplary embodiment, an apparatus that is provided includes the processor device that is further configured to determine the position of the ICE probe with a three-dimensional (3D) volume at a location-oriented in front of the ICE probe; and check the 3D volume of the location-oriented in the front of the ICE probe based on a set of multiple criteria that assess factors associated with the 3D volume, the cardiac anatomy, and the position of the catheter used in the TAVR procedure.

In at least one exemplary embodiment, an apparatus that is provided includes the processor device that is further configured to: determine a 3D sub-volume within the 3D volume wherein the 3D sub-volume comprises a safe-zone and detect a deviation of the ICE probe from the safe-zone configured within the 3D volume wherein the safe-zone is oriented in front of the ICE probe.

In at least one exemplary embodiment, an apparatus that is provided includes the processor device that is further configured to: provide notification via sensory feedback of the deviation of the ICE probe from the safe zone.

In at least one exemplary embodiment, an apparatus that is provided includes the processor device that is further configured to: apply a registration algorithm to determine the 3D orientation of the ICE probe within the cardiac anatomy.

In at least one exemplary embodiment, an apparatus that is provided includes the processor device that is further configured to record a spatial position and the 3D orientation of the ICE probe as a combined reference position; plot a virtual 3D zone by comparing the combined reference position to a location in an X-ray image received by the feedback processor device to determine the safe-zone of the ICE probe; and compare the movement of the ICE probe based on the virtual 3D zone for any deviations of the ICE probe to different locations within the cardiac anatomy.

In another exemplary embodiment, an apparatus to stabilize an Intracardiac Echocardiography (ICE) probe in an endoscopic procedure is provided. The apparatus includes a balloon device disposed about an elongated shaft section at the distal end of the ICE probe at an outer surface for insertion with the distal end of the ICE probe during the endoscopic procedure; an inflatable balloon disposed about a portion of the outer surface at the distal end of the elongated shaft section that is configured in a first state for insertion into the vessel lumen to travel down the vessel lumen, and a second state to stabilize the head of the ICE probe; and in response to the inflatable balloon at the distal end of the elongated shaft section placed at a location about the head of the ICE probe in the vessel lumen, the inflatable balloon is configured to stabilize the head of the ICE probe in the second state by exerting a compression force to the distal end of the ICE probe that includes the head of the ICE probe wherein the compression force is caused by inflation of the inflatable balloon sized to the vessel lumen and pressing against the ICE probe.

In at least one exemplary embodiment, an apparatus that is provided includes the ICE probe stabilized at a position in the vessel lumen to allow for a view of a target cardiac anatomy during the endoscopic procedure.

In at least one exemplary embodiment, an apparatus that is provided includes the inflatable balloon sized to about a diameter of the vessel lumen to stabilize the head of the ICE probe in the second state.

In at least one exemplary embodiment, an apparatus that is provided includes the inflatable balloon of the balloon device with a compliant material that prevents overstretching of the vessel lumen in the second state.

In at least one exemplary embodiment, an apparatus that is provided includes the balloon device to stabilize the ICE probe configured using an occlusion balloon type of device.

In at least one exemplary embodiment, an apparatus that is provided includes in response to the balloon device stabilizing the ICE probe in the vessel lumen, a laser catheter is enabled to perform the ablation for the endoscopic surgery by a single user as further assistance of another user to position the head of the ICE probe to capture the view of the target anatomy during the endoscopic procedure is no longer require.

In at least one exemplary embodiment, an apparatus that is provided includes the balloon device with a compliant material that prevents overstretching of the vessel lumen when the inflatable balloon device is in the second state.

In at least one exemplary embodiment, an apparatus that is provided includes the balloon device configured to prevent movement of the head of the ICE probe in the vessel lumen by the compression force of the distal end of the ICE probe against the vessel lumen.

In at least one exemplary embodiment, an apparatus that is provided includes the balloon device is configured to prevent movement of the head of the ICE probe caused by a set of actions that comprise blood flow, user interaction, and cardiac motion in the vessel lumen.

In yet another exemplary embodiment, a system to assist in a valve resection procedure is provided. The system includes a Neural Network (NN) model to detect and to predict three-dimensional landmarks in a valve resection procedure for proper localization wherein the NN model is a semi-supervised trained NN based on a set of ultrasound anatomical images generated in a prior endoscopic procedure, and a processor device configured to implement the NN model by processing a set of images of cross-sectional views of leaflets in the valve resection procedure to monitor a grasping operation of a leaflet by a grasping mechanism and to provide confirmation of proper leaflet insertion during the grasping operation based on image comparisons of images of leaflet insertions during the grasping operation with cross-sectional views of leaflets contained in the NN model.

In at least one exemplary embodiment, a system that is provided includes the system with the processor device is configured to implement the NN model to track the motion of leaflets during the grasping operation for comparisons of aspects of leaflet motion to determine the proper leaflet insertion.

In at least one exemplary embodiment, a system that is provided includes the ML system with the processor device is configured to implement the NN model to compare pre-grasping leaflet motion versus post-grasp motion to determine the proper leaflet insertion.

In at least one exemplary embodiment, a system that is provided includes the ML system with the processor device is configured to implement the NN model to estimate pre-grasping motion versus post-grasping motion to determine the proper leaflet insertion

Furthermore, other desirable features and characteristics of the system and method will become apparent from the subsequent detailed description and the appended claims, taken in conjunction with the accompanying drawings and the preceding background.

### BRIEF DESCRIPTION OF THE DRAWINGS

The present application will hereinafter be described in conjunction with the following drawing figures, wherein like numerals denote like elements, and:
FIG. 1 illustrates an exemplary diagram of a process of determining a visual view of the anatomical region, positioning of the Intracardiac Echocardiography (ICE) probe, and anchoring of the ICE probe that enables stability of field of view (FOV) displayed on a display device to the medical provider when performing the laser resectioning by the catheter in the endoscopic procedure, in accordance with an embodiment;
FIG. 2 illustrates an exemplary diagram for placement of the ICE probe and a process to estimate the ICE probe position of the ICE probe guidance and placement system in accordance with an embodiment;
FIG. 3. illustrates an exemplary diagram of an inflatable balloon device implemented to position and secure the ICE probe once guided to the optimum viewing position of the ICE probe guidance and placement system in accordance with an embodiment;
In FIG. 4, illustrates an exemplary diagram of the combination of the balloon and the ICE probe at the position with the ICE probe secured once guided to the optimum viewing position of the ICE probe guidance and placement system in accordance with an embodiment;
FIG. 5 illustrates an exemplary diagram of the elasticity of the inflatable balloon of the balloon device used to secure the ICE probe of the ICE probe guidance and placement system in accordance with an embodiment;
FIG. 6, illustrates a diagram of the soft-zone and a desired parking position in the right atrium (RA) of the ICE probe in combination with the laser device in accordance with an embodiment;
FIG. 7 illustrates an exemplary diagram of the desired valve view that triggers a fail-safe process to disable the laser device and provide sensory feedback to the operator in accordance with an embodiment;
FIG. 8 illustrates an exemplary diagram of a common coordinate frame with a reference for navigating an ICE volume with anatomical landmarks in accordance with an embodiment;
FIG. 9 illustrates an exemplary diagram of a combination of a neural network (NN) trained system in multi-task learning for predicting anatomical landmarks in accordance with an embodiment;
FIG. 10 illustrates an exemplary diagram of a Spatial transformer network (STN) to process inputs from the encoder of a localization network in accordance with an embodiment;
FIG. 11 illustrates an exemplary diagram of a machine learning (ML) system for enabling a type of multitask learning network for detecting devices in three-dimensional (3D) volumes in accordance with an exemplary embodiment;
FIG. 12 illustrates an exemplary diagram of a piecewise approximation for combining landmarks from x-ray to ultrasound to improve the distal shape and catheter segmentation of catheter-like devices in accordance with an embodiment;

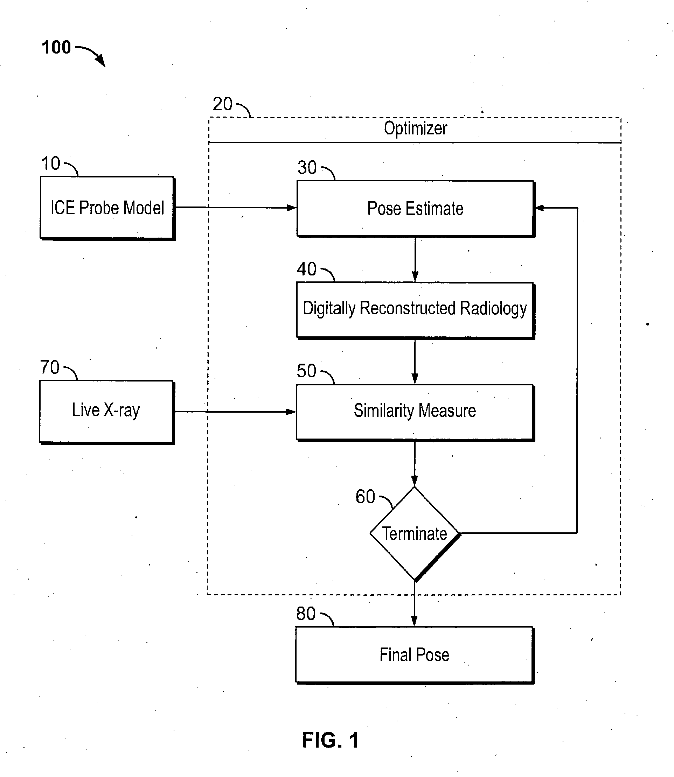

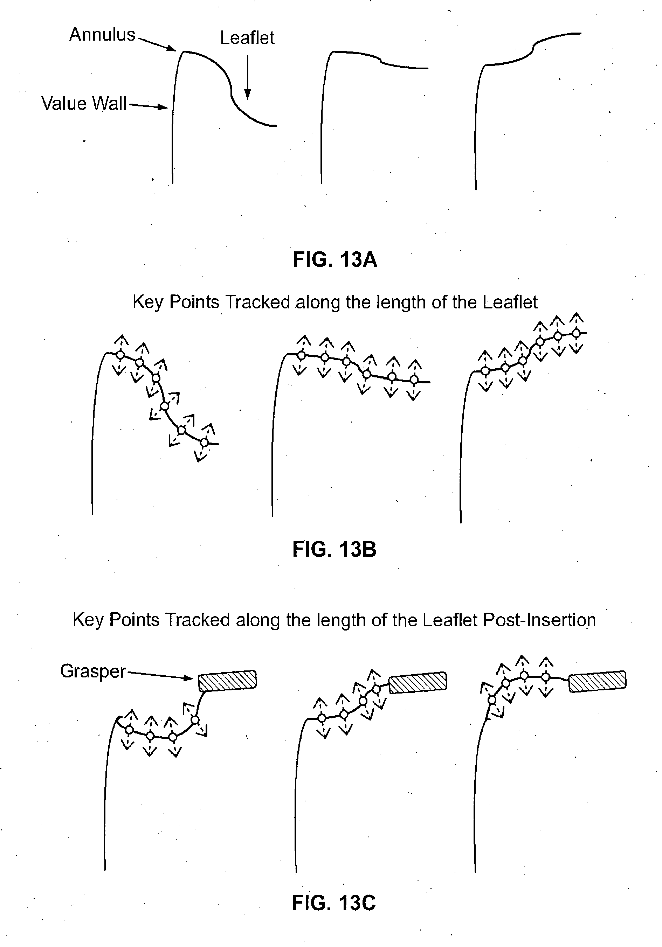

FIGS. 1A, 13B, and 13C illustrate exemplary diagrams of leaflet motion estimations for a leaflet resection procedure in accordance with an embodiment; and

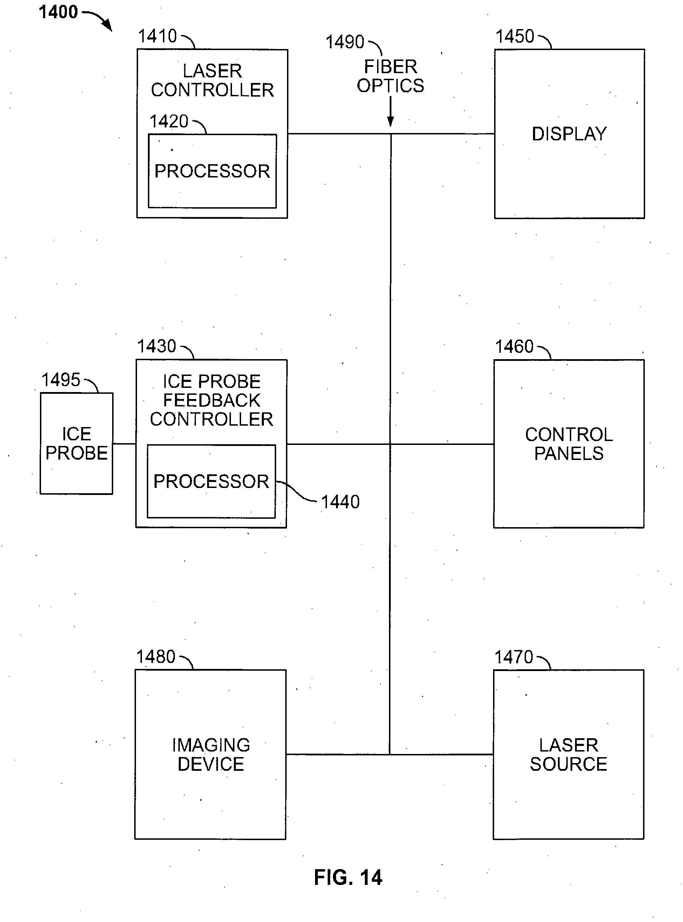

FIG. 14 illustrates a laser ablation system, ICE probe feedback system, and imaging system in accordance with an embodiment.

### DETAILED DESCRIPTION

The following detailed description is merely illustrative in nature and is not intended to limit the embodiments of the subject matter or the application and uses of such embodiments. As used herein, the word "exemplary" means "serving as an example, instance, or illustration." Thus, any embodiment described herein as "exemplary" is not necessarily to be construed as preferred or advantageous over other embodiments.

The embodiments described herein are exemplary embodiments provided to enable persons skilled in the art to make or use the invention and not to limit the scope of the invention that is defined by the claims. Furthermore, there is no intention to be bound by any expressed or implied theory presented in the preceding technical field, background, summary, or the following detailed description.

With increased numbers of cardiac implantable electronic devices (CIEDs) such as pacemakers, defibrillators (ICD), and cardiac resynchronization therapy (CRT) devices in patients, and consistent with the higher numbers of CIED implanted, there is also more complications, infections, and malfunctions by the greater number of CIEDs in use that necessitate more procedures. The TAVR procedure allows for the implanting (i.e., replacing) of a heart valve without having to open the chest cavity. The resultant minimal invasive surgery for a heart valve replacement makes surgical valve replacement are more feasible treatment plan. This is because TAVR can now be considered an option for patients considered at intermediate or high risk of complications from traditional open-chest surgical aortic valve replacement.

However, there are differences in both approaches; for example, in open-chest replacement surgery, the degraded valve is completely removed. In TAVR, the damaged native valve is left in place. The valve can have anatomical abnormalities, calcification, or infection. However, inserting a new valve over the native valve can cause complications in the TAVR procedure, including valve migration, valve embolization, paravalvular leakage, and blockage of the coronary arteries restricting blood flow to the heart.

The TAVR procedure allows for the implanting (i.e., replacing) of a heart valve without having to open the chest cavity. The resultant minimal invasive surgery for a heart valve replacement makes surgical valve replacement are more feasible treatment plan. This is because TAVR can now be considered an option for patients considered at intermediate or high risk of complications from traditional open-chest surgical aortic valve replacement.

Therefore, deploying a TAVR valve on top of the existing damaged valve may not be performed because of the procedure's expected complications. To alleviate the complications from implantation of a new valve, a catheter may be used to remove old valve leaflets at the location to prepare the implant site for a cleaner valve deployment and operation

This present disclosure describes systems and apparatuses that provide the process to stabilize the ICE probe used as an example in a TAVR procedure, with user-based navigation guidance and device trajectories with respect to anatomical landmarks, that facilitate the interaction of the re-sectioning device with its intended target (i.e., the valve leaflet).

In the various exemplary embodiments, the present disclosure describes systems and apparatuses that provide enhance situational awareness of the current spatial location of the ICE probe and for optimum anatomical viewing to improve visualization by the medical provider when performing an endoscopic procedure.

In the various exemplary embodiments, the present disclosure describes systems and apparatuses that provide better stability to the ICE probe head to minimize the effects of cardiac motion that may interfere in performing the endoscopic procedure. Also, once the probe head is stabilized in the desired position, a single provider can determine the three-dimensional (3D) ultrasound (US) volume and manipulate the tip and trajectory of the laser catheter in the 3D US volume.

In the various exemplary embodiments, the present disclosure describes systems and apparatuses that predict a safe zone within the cardiac anatomy for the ICE probe and facilitate the confining for the ICE probe within a defined zone area to ensure smooth execution of the ablation procedure by the laser catheter. Also, if a deviation of the probe from his zone occurs, the deviation can be detected, and the operator is notified via sensory feedback.

While the present disclosure in various embodiments, describes the application of the ICE probe in the context of laser resectioning of aortic valve leaflets, it is contemplated that the implementation described applies to a variety of endoscopic procedures and is not limited to resectioning of aortic valve leaflets. For example, the methodology described is at the very least applicable to the endoscopic procedures directed to the other three valve leaflets in the anatomical region in equal measure and may also apply to a host of other endoscopic type procedures.

For example, the described application of the ICE probe enables an endoscopic procedure to be performed by a single medical provider since the ICE probe once positioned and stabilized can free up the single medical provider to perform the other endoscopic tasks such as the ablation by the laser catheter that ordinary would require an additional set of hands to perform.

The figures and descriptions below provide more detail.

Turning now to FIG. 1 in an embodiment, FIG. 1 illustrates an exemplary diagram of a process of determining a visual view of the anatomical region, a positioning (i.e., parking) of the ICE probe, and anchoring of the ICE probe that enables stability of field of view (FOV) displayed on a display device to the medical provider when performing the laser resectioning by the catheter in the endoscopic procedure, in accordance with an embodiment.

In FIG. 1, positional data is generated by an ICE probe model 10 to an optimizer module 20 to provide instruction of an ICE probe positioning. The positional data generated is sent to a pose estimation module 30. The pose estimation module 30 is also connected to a feedback signal that instructs the pose estimation module 30 whether the results generation is sufficient or whether the process requires additional cycling to refine the results. The feedback signal or signal to terminate the process flow is received from the decision module 60. The pose estimation module 30 generates an output of pose results for the ICE probe to reconstruct module 40. The reconstruct module 40 generates a radiograph or digital reconstruction of the internal structure that is viewed based on the pose estimation results. The similarity module 50 compares the input from a live X-ray module and the output from the digitally reconstructed radiograph to determine the closeness of the digitally reproduced image to an X-ray of the anatomical structure. The decision module 60 determines based on the comparisons of the digitally reproduced image and the X-ray image if the similarity measure between both images is close enough or of a sufficient amount. If the similarity measure is deemed not within a required or sufficient measurement amount, the decision module 60 determines not to terminate the process flow but to return to the pose estimation step and sends a feedback type signal to the pose estimation module 30 to further refine the pose estimation for try for better pose results that are closer to the similarity measurement needed. The process flow returns to the pose estimation module to generate a more refined pose estimation and the comparison in the similarity module is performed again.

The ICE probe is placed directly inside the heart through the vasculature of the body. The position of the probe inside the heart is crucial for a clear view of the target anatomy. The optimal parking position of the ICE probe requires awareness of the probe location in the heart anatomy along with the live view of the anatomy being captured by the imaging elements.

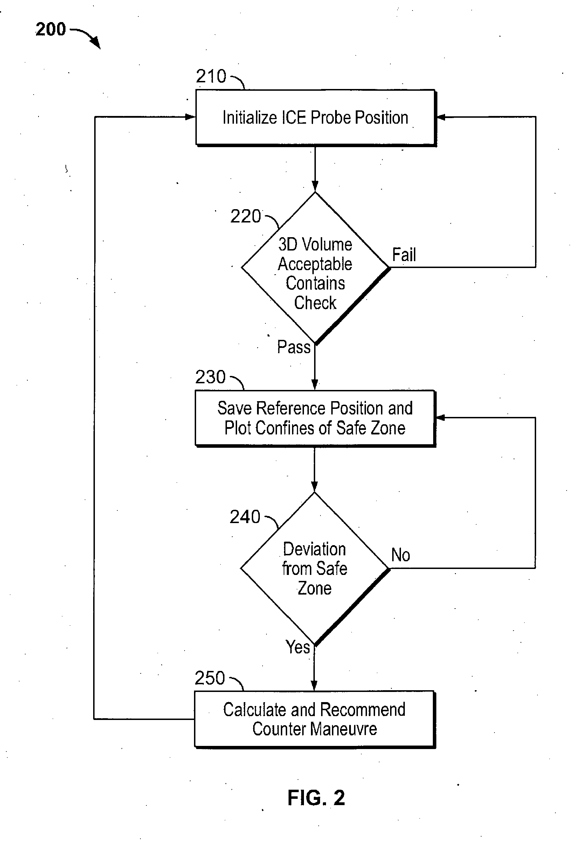

FIG. 2 illustrates an exemplary diagram for placement of the ICE probe and a process to estimate the ICE probe position of the ICE probe guidance and placement system in accordance with an embodiment. In FIG. 2, at the ICE probe initial position 210, the spatial position of the and orientation of the ICE probe is recorded as a reference position for the endoscopic procedure. Next, the system proceeds in the process flow to determine whether the initial location is the optimal placement or whether the ICE probe requires physical movement to obtain a view that admissible for the endoscopic procedure. The optimal placement requires awareness of the ICE probe's current position with the available live views of the anatomy that can be captured by the imaging elements based on the ICE probe placement. At step 220, the three-dimensional (3D) volume is determined surrounding the ICE probe tip by viewing the x-ray image, and acceptance criteria check.

The U. S. Patent Application No. 16/979,542 entitled ULTRASOUND IMAGING PLANE ALIGNMENT GUIDANCE FOR NEURAL NETWORKS AND ASSOCIATED DEVICES, SYSTEMS, AND METHODS assigned to KONINKLIJKE PHILIPS N. V. EINDHOVEN is incorporated by reference and describes a guidance system for obtaining a medical image includes a processor configured to obtain a motion control configuration for repositioning an imaging device from a first imaging position to a second imaging position with respect to a subject's body, the motion control configuration obtained based on a predictive network, an image of the subject's body captured while the imaging device is positioned at the first imaging position, and a target image view including a clinical property; and a display in communication.with the processor and configured to display an instruction, based on the motion control configuration, for operating a control component in communication with the imaging device such that the imaging device is repositioned to the second imaging position.

The volume of the zone is predetermined based on the procedure and must accommodate an acceptable motion. The diameter of the zone is modeled as a sphere-like shape and is computed based on the similarity between the live 3D ultrasound volume and the ideal volume that captures the anatomy of interest (i.e. the leaflet valve) at a favorable viewpoint position. The configured unique zone is therefore centered on the patient-specific anatomy. At step 230, the reference positions are saved as well as the plot confines of the safe zone. At step 240, the deviations are determined from the safe zone. The saved references and plot confines are updated by a feedback loop of input or the deviation information generated at step 240. Once completed, an algorithmic solution is used to calculate and recommend counter maneuvers for the ICE probe placement at step 250. The feedback system to control anchoring system can include additional, utilizing the view finding elements described in FIG. 2, and a manual or autonomous control loop implemented for dynamic anchoring of the system. For example, based on imaging features in x-ray and ultrasound, any deviation of the probe head or loss of view can be used to send signals to the anchoring system and either prompt user to reposition or automatically reposition the probe.

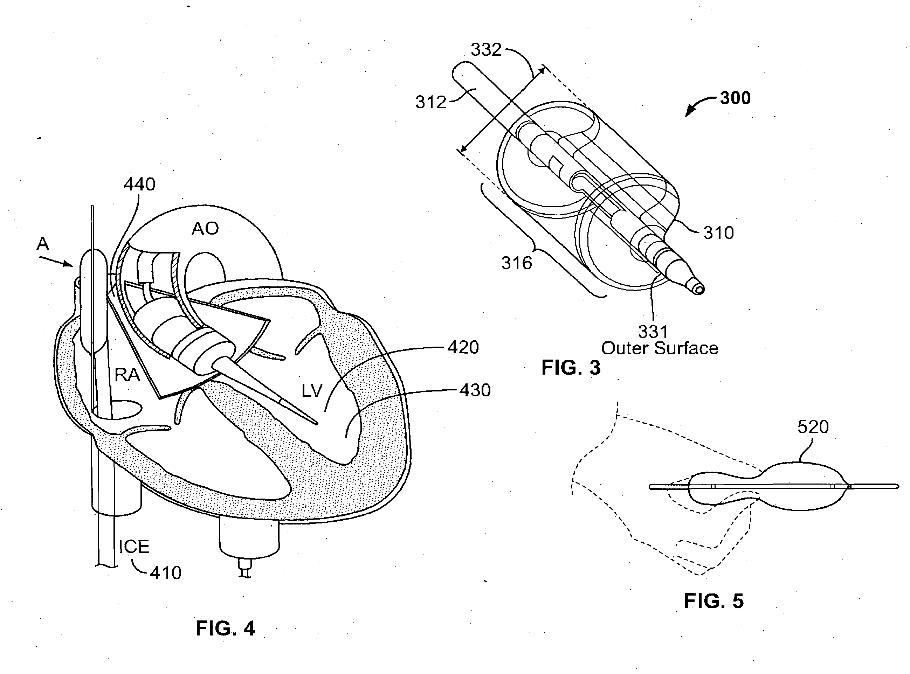

FIG. 3. is a diagram of a balloon catheter system implemented to position and secure the elongated shaft section 312 of an ICE probe 316 once guided to the optimum viewing position by the ICE probe guidance and placement system in accordance with an embodiment. In FIG. 3, the inflatable balloon 310 can be configured to form an envelope around the ICE probe 316 to secure the ICE probe in a position for the optimum viewing of the valve leaflet. The inflatable balloon 310 is configured to not impede the flow of blood by enabling blood flow through a secondary lumen that can be integrated into the ICE probe or formed when the balloon is expanded in the lumen.

In an exemplary embodiment, the ICE probe 316 is stabilized in a manner to secure the position of the ICE probe 316 in the lumen and at a position that minimizes tremors that can be realized by the probe tip and are caused by internal body cavity vibrations (or movements) from a beating heart. Upon a determination or discovery of the optimum, correct, or best available FOV in the lumen (i.e., the superior vena cava (SVC) lumen), the stabilizing process for the ICE probe would include a compliant inflatable balloon 310 sized to the diameter 332 of the SVC, then placed or integrated at a section or part (i.e., the elongated shaft section 312) near the ICE probe head and the balloon would be configured in an inflated state. The ICE imaging window may require adjustment after inflatable balloon inflation because of any interference caused by the expanded state of the balloon.

In an exemplary embodiment, the balloon device 300 is placed around an elongated shaft section 312 of the ICE probe 316 that has an outer surface 331 for insertion at a distal end of the ICE probe 316 with the elongated shaft section 312 into a vessel lumen during the endoscopic procedure. The inflatable balloon 310 is located and disposed of about a portion of the outer surface 331 at the distal end of the elongated shaft section 312. The inflatable balloon 310 is placed in a first state for insertion into the vessel lumen to travel down the vessel lumen and placed in a second state to stabilize the head of the ICE probe. The inflatable balloon 310 when inflated in the second state can exert a compression force to the distal end of the ICE probe that includes the head of the ICE probe 316.

The compression force is caused by inflation of the inflatable balloon sized to the vessel lumen and pressing against the ICE probe 316. The inflatable balloon 310 is sized to about a diameter of the vessel lumen to stabilize the head of the ICE probe 316 in the second state. The inflatable balloon 310 is made of a compliant material that prevents overstretching of the vessel lumen in the second state. The balloon device 300 is configured using an occlusion balloon type of device. With the ICE probe 316 stabilized, a single user can perform the procedure because no assistance is required to manually hold or position the ICE probe 316 when using the laser catheter to perform the ablation. An example of a legacy product of an occlusion balloon is the PHILIPS^{®} BRIDGE^{™} Occlusion balloon.

In Fig. 4, is an exemplary diagram of the combination of the balloon and the ICE probe at the position with the ICE probe secured once guided to the optimum viewing position of the ICE probe guidance and placement system in accordance with an embodiment. In FIG. 4, the ICE probe and balloon configuration 410 is shown with the ICE probe snaked to a correct viewing location through the lumen and the tip 420 in a volumetric space 430 to enable the visual image of the leaflet display. The compression of the ICE distal tip 440 secures in the lumen and a placement location prevents the ICE displacement and motion that can happen from the blood flow, the medical provider interaction, or cardiac motion (i.e., the vibrations caused by internal heartbeats).

In FIG. 5, is a diagram that displays the elasticity of the balloon used to secure the ICE probe of the ICE probe guidance and placement system in accordance with an embodiment. The balloon 510 is configured using a compliant, stretchable, and mouldable material, that when expanded is limited in size to prevent an overstretching of the SVC lumen of the puncture from a reaction force caused by compressing the ICE distal tip into the lumen or heart walls. An example of an inflatable balloon is the PHILIPS^{®} Bridge Occlusion balloon. In an exemplary embodiment, the balloon device 300 can be configured in 80 mm in length and 20 mm in diameter with a maximum inflation volume of 60 cc. The U.S. Patent No. 10,499,892 entitled TEMPORARY OCCLUSION BALLOON DEVICES AND METHODS FOR PREVENTING BLOOD FLOW THROUGH A VASCULAR PERFORATION, filed March 16, 2016, and issued on December 10^{th}, 2019 assigned to SPECTRANETICS^{®} Corporation with inventors Ryan Michael Sotak, Grant Foy, Phil Aranas, Jay Harper is incorporated by reference.

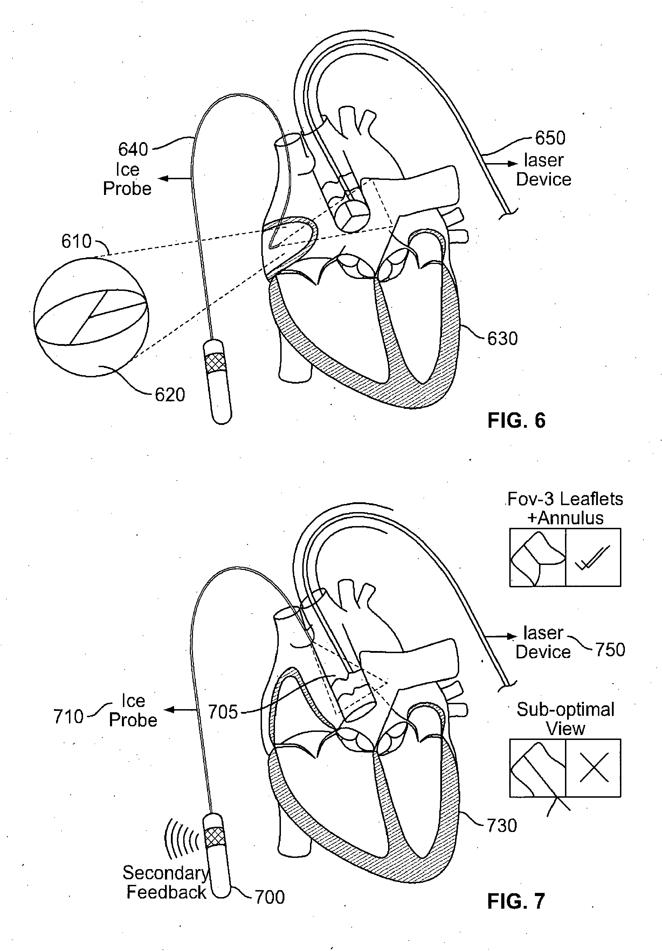

FIG. 6, illustrates a diagram of the soft zone and a desired parking position in the right-atrium (RA) of the ICE probe 640 in combination with the laser device in accordance with an embodiment. The laser device 650 is positioned in the right atrium 630, with a diameter 610 and view 620.

FIG. 7 illustrates a desired valve view that triggers a fail-safe process to disable the laser device and provide sensory feedback to the operator, in accordance with an embodiment. The laser device 750 and grasping mechanism 705 is coupled with the ICE probe 710 with a field of view in the right atrium 730 for the soft zone. The failsafe can be triggered because of a deviation of the ICE probe 710 from an anchored position or loss of view based on positional comparisons with the X-ray. For example, based on imaging features in x-ray and ultrasound, any deviation of the probe head or loss of view can be used to send signals to the anchoring system and either prompt user to reposition or automatically reposition the probe.

In an exemplary embodiment, the sensory feedback device 700 includes a processor device to implement a machine learning (ML) algorithm or system to assist in a valve resection procedure. The ML algorithm can apply a Neural Network (NN) model to detect and predict three-. dimensional landmarks in a valve resection procedure for proper localization. The NN model may be configured as a semi-supervised trained NN based on a set of ultrasound anatomical images generated in a prior valve resection procedure. In an exemplary embodiment, the sensory (processor) feedback device 700 can implement the NN model by processing a set of images of cross-sectional views of leaflets in the valve resection procedure to monitor a grasping operation of a leaflet by a grasping mechanism 705 and to provide confirmation of proper leaflet insertion during the grasping operation based on image comparisons of images of leaflet insertions during the grasping operation with cross-sectional views of leaflets contained in the NN model. The ML system with the processor device is configured to implement the NN model to track the motion of leaflets during the grasping operation for comparisons of aspects of leaflet motion to determine the proper leaflet insertion. The ML system with the processor device is configured to implement the NN model to compare pre-grasping leaflet motion versus post-grasp motion to determine the proper leaflet insertion. The ML system with the processor device is configured to implement the NN model to estimate pre-grasping motion versus post-grasping motion to determine the proper leaflet insertion.

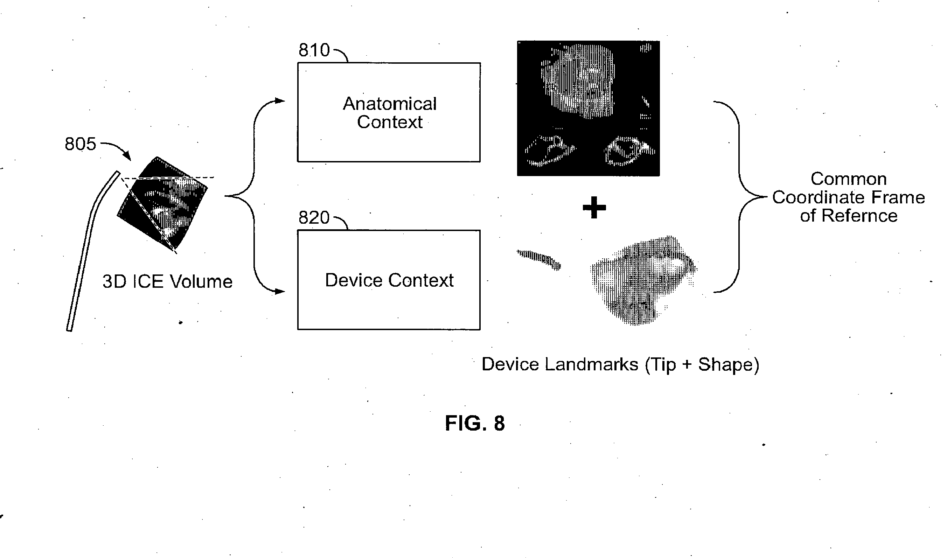

FIG. 8 illustrates a diagram of a common coordinate frame with a reference for navigating an ICE volume with anatomical landmarks in accordance with an embodiment. FIG. 8 illustrates a common coordinate frame of a reference to navigate in an ICE probe configured volume area with anatomical landmarks. In FIG. 8, processing module 810 provides an anatomical context with the anatomical landmarks and the common coordinate frame of reference. The processing module 820 provides the ICE probe context in the 3D ICE probe volume 805 for navigation of the laser catheter

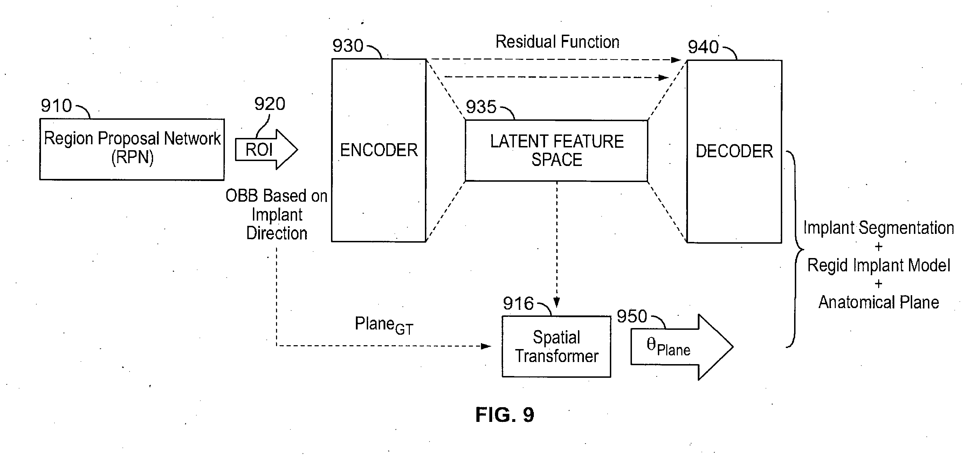

FIG. 9 illustrates a diagram of a combination of a neural network (NN) trained system in multi-task learning for predicting anatomical landmarks in the resection procedure in accordance with an embodiment. FIG. 9 includes a region proposal network (RPN) 910, that configures a region for interest 920 for an Opaque Binary Blob (OBB) based on the determined implant direction and is sent to an encoder 930 to determine the latent feature space 935. This feature space is received by the decoder 940 that performs a series of functions that can include implant segmentation, rigid implant modeling, and anatomical plane configuration. Also, planar transforms are configured by the planar transformer 950 for displaying the two-dimensional (2D), 3D, and four-dimensional (4D) ultrasound images. The algorithm controller is designed to detect, localize, and classify valve and surrounding anatomy in a 3D ultrasound image to ensure the proper leaflet resection. The system is capable of receiving a set of 3D and time ultrasound images, receiving ground truth, manual expert annotations for key-points of valve anatomy and about labels associated with anatomy, and automated key-points and labels using a local fitting deformable geometric model. In an exemplary embodiment, the training of a machine learning model or neural network model using 3D ultrasound images to detect and predict 3D locations of anatomical landmarks, regress anatomical valve plane, generate candidate segmentation for fitting anatomical valve model, etc is implemented. This implementation may be in the form of a cascade of connected neural network models which are designed to execute the training of each neural network model in a semi-supervised multi-task learning paradigm.

In an exemplary embodiment, to specifically localize the valve, a region proposal network can be trained to detect and localize the subset of the image containing the valve. The output of the network is an oriented bounding box, the extent of which can be defined by the geometric limits of the valve or implant model. This localized sub-volume (SV) is then processed by an encoder-decoder network configuration for multi-task learning of latent space vectors which can be used to reconstruct a probabilistic segmentation and also to train a spatial transformer for regressing a plane containing the anatomical reference (e.g., valve plane showing the cross-section of leaflets). The spatial transformer is also trained to generate a transformation matrix that can define a 2D plane used to sample the 3D sub-volume to produce an anatomical plane. This is training is performed by optimizing a loss function that minimizes the structural similarity index loss and means squared loss between the generated plane and ground truth plane sampled from labeled data. The ground truth images generated are based on standard 2D US images and the ground truth images can be added to the training process to improve the trained model. The network when applied can generate an anatomical plane of relevance and a candidate sparse segmentation. This information can reduce the burden of a large amount of labeled data. A combination of the anatomical plane and sparse segmentation mask can be used to initialize a deformable or rigid image-based model-fitting algorithm local anatomical coordinate frame of reference.

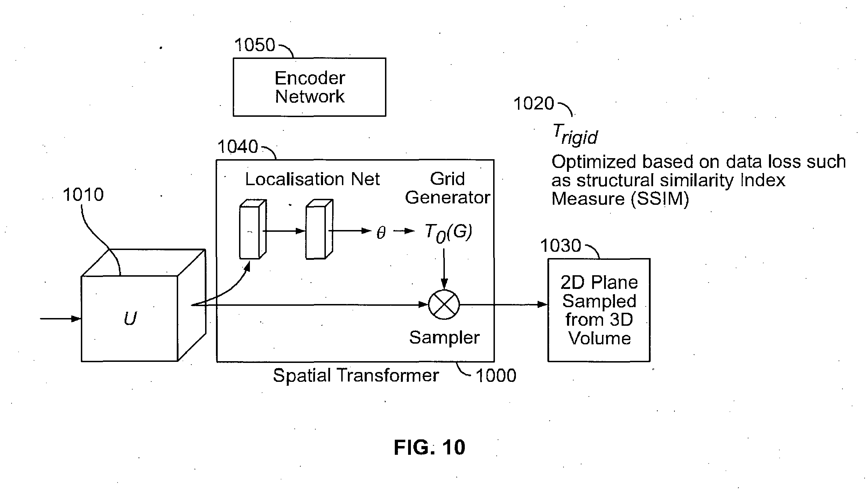

In FIG. 10 an exemplary diagram of a Spatial transformer network (STN) 1000 is illustrated that receives and consumes inputs from the encoder that defines a localization network. The latent feature vector generated by this process is condensed to regress a rigid transformation matrix T_{rigid} 1020. The transformation matrix is used to transform a 2D identity plane within the span of the input 3D volume 1010. The plane generated is then applied to sample a 2D slice from the 3D volume. The STN 1000 is trained based on the global multi-task loss which includes image data loss based on metrics such as structural similarity index. The trained STN is capable to generate a 4x3 transformation matrix T_{rigid} 1020 which defines a transformation 1040 to be applied to a 2D plane used to sample the 3D sub-volume to produce an anatomical plane 1030. The initial transformation 1040 is set as an identity matrix located at the center of the 3D sub-volume. The identity matrix is updated by optimizing a loss function that minimizes the structural similarity index loss and means squared loss between the generated plane and ground truth plane sampled from labeled data. Iteratively the (encoder) network 1050 trains to regress the pose of a 2D plane to output a pose that is closest and resembles the anatomic plane of interest from the 3D volume. Additionally, ground truth images received from standard 2D US images can be added to the training process. The network 1050 learns to generate an anatomical plane of relevance and a candidate sparse segmentation. This reduces the burden of a large amount of labeled data. A combination of the anatomical plane and sparse segmentation mask can be used to initialize a deformable or rigid image-based model-fitting algorithm local anatomical coordinate frame of reference.

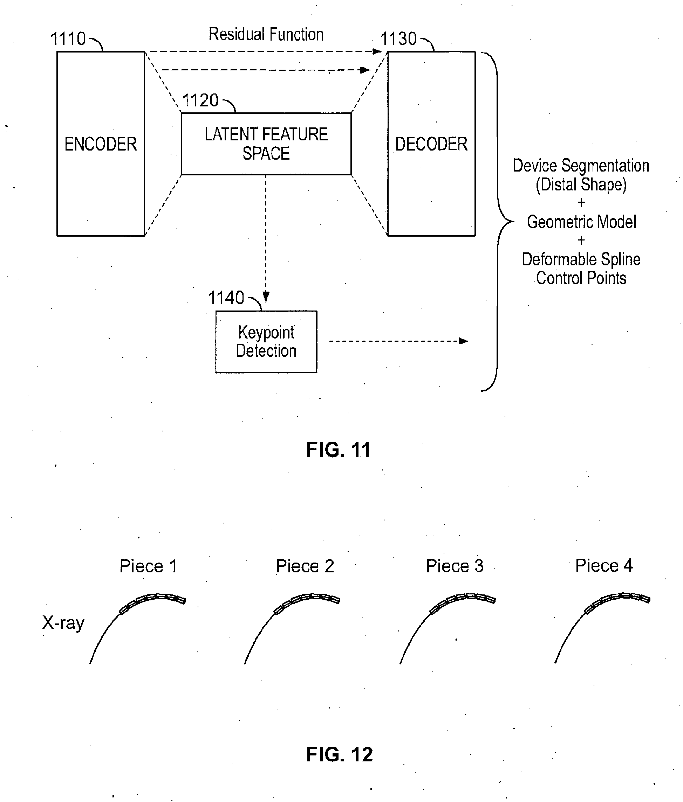

FIG. 11 illustrates an exemplary diagram of a machine learning system for enabling a type of multitask learning network for detecting devices in 3D ultrasound volumes, in accordance with an exemplary embodiment. The system includes an encoder 1110, a latent feature space 1120, a decoder 1130, and a key point detection module 1140 that enable device segmentation of the distal shape with a geometric model and the use of deformable spline control points.

The training of a machine learning model or neural network model using 3D ultrasound images can detect and predict 3D landmarks for non-anatomical features such as implants using co-registered 3D ultrasound and 2D x-ray data. The catheter-like devices often appear ambiguous in ultrasound images and can be misinterpreted for anatomical features when the devices are positioned closer to tissue. In a dynamic environment that is present during the tissue resection, discerning the ultrasound images can become even more challenging. An example of setting device tip location and distal end shape is to train a network for the multi-task learning paradigm. This training is performed either by fully supervised in a semi-supervised fashion where data labeling constraints are more relaxed. The typical architectures used for this are an Encoder-Decoder combination where convolutional filter banks decompose the input image into a feature vector that captures the minimum information required to extract the desired feature. In this multi-task learning paradigm, the latent features are then used to train a key point detection network, such as a 3D Yolo or convolution pose machines, which can provide potential candidate locations of the central axis of the catheter in ultrasound. The decoder part of the network is used to also reconstruct the learned features back up to the scale of the original image in the form of a probability map. This can be used to reconstruct a potential segmentation map of the image.

FIG. 12 illustrates an exemplary diagram of a piecewise approximation for combining landmarks from x-ray to ultrasound to improve the distal shape and catheter segmentation of catheter-like devices in accordance with an embodiment.

A critical part of the resection procedure is proper localization and grasping of the targeted leaflet. The localization of the leaflet is done as per the methods described in the previous section. Once this is done, the leaflet is imaged in an orthogonal cross-sectional view where grasping is monitored for proper leaflet insertion in the grasping mechanism. As a confirmation of proper leaflet insertion, the motion of the leaflet is tracked in the cross-sectional 2D imaging plane utilizing methods such as speckle tracking. Comparing pre-grasping leaflet motion via piecemeal portions in a series of parametric time-based models, particularly with respect to the tip of the leaflet, versus post-grasp motion can aid in determining the quality of insertion providing the clinician with the necessary confidence in advancing the laser re-sectioning device. In FIG. 12 the series of piecemeal approximations (Piece 1-4) of the movement of the ICE probe or other catheter-like devices can be fitted together to monitor the localization of the grasping operations in the leaflet ablation processes for the endoscopic procedure.

In an exemplary embodiment, the segmentation maps, as well as key point detection, tend to be noisy. But having this spatial information, it is possible to reduce the search space for local model-to-image-based registration techniques. Additionally, in X-ray imaging, the above-described network architecture can be utilized to perform keypoint detection, shape segmentation, and 3D pose regression based on training networks using synthetic images generated from DRR. By utilizing the known spatial relationship between ICE volume and corresponding X-ray image, the detection of device key points is projected along epipolar lines back to ultrasound space, the search space for geometric model localization is reduced. The information from X-ray space is then combined with ultrasound in a piecewise fashion which allows the model fitting to be a 3D parametric spline fitting problem. Additional contextual constraints can be added to the geometry of the device. For example, catheter-like devices often have radio-opaque markers that are spaced at a certain distance from the tip. This can add to improving the accuracy of the tip localization in ultrasound space.

FIGS. 2A, 13B, and 13C illustration exemplary diagrams of leaflet motion estimations for a leaflet resection procedure in accordance with an embodiment. In FIG. 13A there is illustrated a leaflet state over time; In FIG. 13B there is illustrated the motion of key points along the length of the leaflet and in FIG. 13C there is illustrated the restricted motion of leaflets after grasping by a tool. The processing unit (in FIG. 7) is capable of performing a series of steps in FIGS. 13A-C that includes accepting ultrasound images (2D; 3D, 2D + time, 3D + time) and executing conventional data (image, signals) processing algorithms and/or machine learning and deep learning algorithms.

In an exemplary embodiment, an algorithmic block applied by the processing unit is configured to detect leaflet key points, track leaflet key points in time, calculate leaflet length, analyze leaflet displacement, etc. The algorithm is designed to localize key features along the length of the leaflet in a 2D/3D cross-sectional view of the valve leaflet. Example of techniques which may be used, but not limited to, are SURF, SIFT, ORB, BRIK, MSER, HARRIS, etc. The key points can be localized in either single or multiple parallels or oblique imaging planes. For robustness, the detected key points can be clustered and resampled. The key points are localized in multiple frames along the time axis (e.g., previous, current, next). Next, the displacement of these key points is calculated between frames. The displacement may be calculated by treating this as a non-rigid image-to-image registration problem between subsequent frames, propagating through time. Owing to the repetitive cardiac motion, the average displacement of the leaflet can be calculated within a few cycles. Methods such as demon registration and its variation, sparse demons can be used for this purpose.

In an exemplary embodiment, a critical part of the resection procedure is proper localization and grasping of the targeted leaflet. The localization of the leaflet is done as per the methods described in the previous section. Once completed, the leaflet is imaged in an orthogonal cross-sectional view where grasping is monitored for proper leaflet insertion in the grasping mechanism. As a confirmation of proper leaflet insertion sent to a feedback device in FIG. 7), the motion of the leaflet is tracked in the cross-sectional 2D imaging plane utilizing methods such as speckle tracking. Comparing pre-grasping leaflet motion, particularly the tip of the leaflet, versus post-grasp motion can help determine the quality of insertion providing the clinician with the necessary confidence in advancing the laser re-sectioning device.

In an exemplary embodiment, in an alternate data-driven approach, a machine learning or neural network model can be trained to detect key points along with the leaflet in a frame by frame manner and then characterize the motion of key points between the frame. Examples of such a network are Yolo or convolutional pose machines. Typically, such methods require supervised training to perform well. In medical imaging, obtaining annotated data is challenging and is typically scarce depending on the modality in question. To overcome this, leaflet motion can be characterized by calculating the flow vector by employing neural models that can be trained in an unsupervised fashion.

The data used for training this network can be loops of leaflet motion within a cardiac cycle without grasping as well as with grasping. Here the neural network learns to generate flow vectors that map two temporally separated images to each other. The network backbone includes an encoder-decoder architecture that decomposes the input image to latent feature space and then reconstructs flow vectors. Two branches of the network are used with weight sharing between two temporally separated images. The network is trained using a combination of loss functions which enforce smoothness of the flow fields, and structural similarity between the pair of images after the new image is warped to the previous image using the flow field vectors. At inference time, the magnitude of the flow field vectors can be used to characterize before and after grasping leaflet motion.

In the implementation, a hybrid approach combining classical (a) and data-driven (b) can be used. Additionally, the cross-sectional 2D and 3D images can be sampled at multiple locations to improve the robustness of characterization. Additionally, flow and hemodynamic information can also be combined with image features to characterize the leaflet grasping and provide a confidence score. Additionally, during data acquisition for the training of the neural network, associated events - (1) grasping successfully and re-grasping for unsuccessful grasps and (2) depth of grasp/leaflet insertion can be tagged. This can be an additional input to classify flow vectors as grasped vs not grasped as well as providing metrics for the level of insertion and confidence.

The application phase controller block is configured to Receive 2D/3D + time ultrasound images, to calculate/Predict flow vectors and magnitude of leaflet motion, to predict the level of insertion of leaflet after grasping as confidence map or a numerical range. The successful evaluation of resection can be visualized by providing the ability to toggle a rendering overlay of the geometric model of the implanted valve as localized in the previous section over a live ICE image.

FIG. 14 illustrates a laser ablation, ICE probe feedback system, and imaging system 1400 according to some embodiments. Laser ablation controller 1410, ICE probe feedback system 1430, an imaging system 1480 which includes a laser generator 1470 coupled to a laser ablation controller 1410. The Controller 1410 includes one or more computing devices having a computer-readable medium programmed to control laser generator 1470, as described in the previous FIGS. 1-13. The controller 1410 may be internal or external to laser generator 1470. Laser generator 1470 may include an excimer laser or another suitable laser. In some embodiments, laser generator 130 produces light in the ultraviolet frequency range. The laser generator 1470 is connected with the proximal end of a laser catheter via a coupler. The distal end of the catheter may be inserted into a vessel or tissue of a human body. In some embodiments, system 1400 employs an ICE probe to guide laser light from the laser generator through a catheter toward a target area in the human body (i.e. target cardiac anatomy). Fiber optic bundle 1490 includes any suitable number of optical fibers and, in some embodiments, to provide coupling between the various controller and devices in use.

The ICE probe feedback controller 1430 includes a processor 1440 with processing capability sufficient to implement various machine learning algorithms and process input data from an imaging device 1480. In addition, controller 1430 provides aural, audible, or other notifications of guidance as an ICE probe 1495 travels down a vessel lumen to a position that provides an optimal field of view of the cardiac autonomy (i.e., leaflets) during the tissue ablation.

Those of skill in the art will appreciate that the various illustrative logical blocks, modules, circuits, and algorithm steps described in connection with the embodiments disclosed herein may be implemented as electronic hardware, computer software, or combinations of both Some of the embodiments and implementations are described above in terms of functional and/or logical block components (or modules) and various processing steps.

However, it should be appreciated that such block components (or modules) may be realized by any number of hardware, .software, and/or firmware components configured to perform the specified functions. To clearly illustrate the interchangeability of hardware, various illustrative components, blocks, modules, and steps have been described above generally in terms of their functionality. Whether such functionality is implemented as hardware or software depends upon the application and design constraints imposed on the overall system.

Skilled artisans may implement the described functionality in varying ways for each application, but such implementation decisions should not be interpreted as causing a departure from the scope of the present invention. In addition, those skilled in the art will appreciate that the embodiments described herein are merely exemplary implementations.

In this document, relational terms such as first and second, and the like may be used solely to distinguish one entity or action from another entity or action without necessarily requiring or implying any actual such relationship or order between such entities or actions. Numerical ordinals such as "first," "second," "third," etc. simply denote different singles of a plurality and do not imply any order or sequence unless specifically defined by the claim language. The sequence of the text in any of the claims does not imply that process steps must be performed in a temporal or logical order according to such sequence unless it is specifically defined by the language of the claim. When "or" is used herein, it is the logical or mathematical or, also called the "inclusive or." Accordingly, A or B is true for the three cases: A is true, B is true, and A and B are true. In some cases, the exclusive "or" is constructed with "and;" for example, "one from the set A and B" is true for the two cases: A is true, and B is true.

Furthermore, depending on the context, words such as "connect" or "coupled to" used in describing a relationship between different elements do not imply that a direct physical connection must be made between these elements. For example, two elements may be connected to each other physically, electronically, logically, or in any other manner, through one or more additional elements.

While at least one exemplary embodiment has been presented in the foregoing detailed description of the invention, it should be appreciated that a vast number of variations exist. It should also be appreciated that the exemplary embodiment or exemplary embodiments are only examples and are not intended to limit the scope, applicability, or configuration of the invention in any way. Rather, the foregoing detailed description will provide those skilled in the art with a convenient road map for implementing an exemplary embodiment of the invention. It is understood that various changes may be made in the function and arrangement of elements described in an exemplary embodiment without departing from the scope of the invention as set forth in the appended claims.

## Claims

1. An apparatus comprising an Intracardiac Echocardiography (ICE) probe for use in a Transcatheter Aortic Valve Replacement (TAVR) procedure, the apparatus comprising:
the ICE probe; and
a processor device coupled to the ICE probe to provide positional feedback to a user about an ICE probe position as the ICE probe is positioned manually within cardiac anatomy;
wherein the processor device is configured to implement a model to provide guidance to manually position the ICE probe based on anatomical recognition of the cardiac anatomy wherein a manually positioned ICE probe is located at a position in the cardiac anatomy to enable capture of a view of a target cardiac anatomy in combination with use of a catheter used while performing the TAVR procedure.

2. The apparatus of claim 1, further comprising:
the processor device is further configured to:
determine an initial position and a three-dimensional (3D) orientation of the ICE probe within the cardiac anatomy to display a field of view(FOV) of the cardiac anatomy while performing the TAVR procedure.

3. The apparatus of claim 2, further comprising:
the processor device is further configured to:
determine the position of the ICE probe with a three-dimensional (3D) volume at a location-oriented in front of the ICE probe; and
check the 3D volume of the location-oriented in the front of the ICE probe based on a set of multiple criteria that assess factors associated with the 3D volume, the cardiac anatomy, and the position of the catheter used in the TAVR procedure.

4. The apparatus of claim 3, further comprising:
the processor device is further configured to:
determine a 3D sub-volume within the 3D volume wherein the 3D sub-volume comprises a safe-zone; and
detect a deviation of the ICE probe from a safe zone configured within the 3D volume wherein the safe zone is oriented in front of the ICE probe.

5. The apparatus of claim 4, further comprising:
the processor device is further configured to:
provide notification via sensory feedback of the deviation of the ICE probe from the safe zone.

6. The apparatus of claim 5, further comprising:
the processor device is further configured to:
apply a registration algorithm to determine the 3D orientation of the ICE probe within the cardiac anatomy.

7. The apparatus of claim 6, further comprising:
the processor device is further configured to:
record a spatial position and the 3D orientation of the ICE probe as a combined reference position;
plot a virtual 3D zone by comparing the combined reference position to a location in an X-ray image received by the feedback processor device to determine the safe zone of the ICE probe; and
compare movement of the ICE probe based on the virtual 3D zone for any deviations of the ICE probe to different locations within the cardiac anatomy.

8. An apparatus to stabilize an Intracardiac Echocardiography (ICE) probe in an endoscopic procedure, the apparatus comprising:
a balloon device disposed about an elongated shaft section at a distal end of the ICE probe at an outer surface for insertion with the distal end of the ICE probe during the endoscopic procedure;
an inflatable balloon disposed about a portion of the outer surface at the distal end of the elongated shaft section that is configured in a first state for insertion into a vessel lumen to travel down the vessel lumen, and a second state to stabilize a head of the ICE probe; and
in response to the inflatable balloon at the distal end of the elongated shaft section placed at a location about the head of the ICE probe in the vessel lumen, the inflatable balloon is configured to stabilize the head of the ICE probe in the second state by exerting a compression force to the distal end of the ICE probe that includes the head of the ICE probe wherein the compression force is caused by inflation of the inflatable balloon sized to the vessel lumen and pressing against the ICE probe.

9. The apparatus of claim 8, further comprising:
wherein the ICE probe is stabilized at a position in the vessel lumen to allow for a view of a target cardiac anatomy during the endoscopic procedure.

10. The apparatus of claim 9, further comprising:
wherein the inflatable balloon is sized to about a diameter of the vessel lumen to stabilize the head of the ICE probe in the second state.

11. The apparatus of claim 10, further comprising:
wherein the inflatable balloon of the balloon device comprises a compliant material that prevents overstretching of the vessel lumen in the second state.

12. The apparatus of claim 11, further comprising:
wherein the balloon device to stabilize the ICE probe is configured using an occlusion balloon type of device.

13. The apparatus of claim 12, further comprising:
in response to the balloon device stabilizing the ICE probe in the vessel lumen, a laser catheter is enabled to perform ablation for the endoscopic procedure by a single user as further assistance of another user to position the head of the ICE probe to capture the view of the target anatomy during the endoscopic procedure is no longer require.

14. The apparatus of claim 8, further comprising:
wherein the balloon device comprises a compliant material that prevents overstretching of the vessel lumen when the inflatable balloon device is in the second state.

15. The apparatus of claim 9, further comprising:
wherein the balloon device is configured to prevent movement of the head of the ICE probe in the vessel lumen by the compression force of the distal end of the ICE probe against the vessel lumen.

16. The apparatus of claim 10, further comprising:
the balloon device is configured to prevent movement of the head of the ICE probe caused by a set of actions that comprise blood flow, user interaction, and cardiac motion in the vessel lum en.

17. A system to assist in a valve resection procedure comprising:
a Neural Network (NN) model to detect and to predict a plurality of three-dimensional landmarks in a valve resection procedure for proper localization wherein the NN model is a semi-supervised trained NN based on a set of ultrasound anatomical images generated in a prior valve resection procedure; and
a processing device configured to implement the NN model by processing a set of images of cross-sectional views of leaflets in the valve resection procedure to monitor a grasping operation of a leaflet by a grasping mechanism and to provide confirmation of proper leaflet insertion during the grasping operation based on image comparisons of images of leaflet insertions during the grasping operation with cross-sectional views of leaflets contained in the NN model.

18. The system of claim 17, further comprising:
the processor device is configured to implement the NN model to track movement of leaflets during the grasping operation for comparisons of aspects of leaflet motion to determine the proper leaflet insertion.

19. The system of claim 18, further comprising:
the processor device is configured to implement the NN model to compare pre-grasping leaflet motion versus post-grasp motion to determine the proper leaflet insertion.

20. The system of claim 19, further comprising:
the processor device is configured to implement the NN model to estimate pre-grasping motion versus post-grasping motion to determine in advance the proper leaflet insertion.
